# EUROPEAN PATENT SPECIFICATION

(11) **EP 2 696 861 B1**
(45) Date of publication and mention of the grant of the patent: **26.09.2018**
(21) Application number: 12717906.7
(22) Date of filing: 16.04.2012
(51) Int. Cl.: A61P 35/00, A61K 31/198, A61K 31/202, A23L 33/12, A23L 33/175

(54) **COMBINATION OF EPA, DPA AND/OR DHA WITH A CHEMOTHERAPEUTIC AGENT**
KOMBINATION VON EPA, DPA UND/ODER DHA MIT EINEM CHEMOTHERAPEUTIKUM
COMBINAISON D'EPA, DE DPA ET/OU DE DHA AVEC UN AGENT CHIMIOTHÉRAPEUTIQUE

(30) Priority: 14.04.2011 WO PCT/NL2011/050255
(43) Date of publication of application: 19.02.2014
(73) Proprietor: N.V. Nutricia, 2712 HM Zoetermeer (NL)
(72) Inventor: DIJK, Francina Jeannette, NL-6871 EX Renkum (NL); VAN NORREN, Klaske, NL-6871 LP Renkum (NL); VAN DIJK-OTTENS, Miriam, NL-4197 HC Buurmalsen (NL)
(74) Representative: V.O.
(86) International application number: PCT/NL2012/050250
(87) International publication number: WO 2012/141590

(56) References cited:
- WO-A1-99/44600
- WO-A1-2006/067498
- WO-A1-2009/157759
- WO-A1-2010/151816
- WO-A2-01/17517
- BARBER M D ET AL: "TOLERANCE AND INCORPORATION OF A HIGH-DOSE EICOSAPENTAENOIC ACID DIESTER EMULSION BY PATIENTS WITH PANCREATIC CANCER CACHEXIA", LIPIDS, SPRINGER, US, vol. 36, no. 4, 1 April 2001 (2001-04-01), pages 347-351, XP009017174, ISSN: 0024-4201, DOI: DOI:10.1007/S11745-001-0726-4

## Description

The present invention is directed to a combination of eicosapentaenoic acid (EPA), docosapentaenoic acid (DPA), and/or docosahexanoic acid (DHA), and EPA, DPA, and/or DHA and a protein, a carbohydrate, a lipid, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, respectively, with a chemotherapeutic agent for use in treating a neoplastic disease. The invention further refers to a method for treating a neoplastic disease comprising administering EPA, DPA, and/or DHA or EPA, DPA, and/or DHA and a protein, a carbohydrate, a lipid, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme. The invention further refers to a composition comprising or consisting of EPA or DPA, preferably treating a neoplastic disease in particular by enhancing the effect of an anti-neoplastic treatment, e.g., enhancing the effect of a chemotherapeutic agent and/or a radiotherapy.

### Background

The improvement of treatment of neoplastic diseases has still high significance as the number of people suffering from neoplastic diseases will further increase in the coming years, particularly due to the aging populations. Even if survival rates have improved dramatically over the past few decades in particular in children and younger patients, there is still an essential need in improving efficacy of the treatment of tumorpatients. In general four standard methods of treatment for cancer exist, which are surgery, chemotherapy, radiation therapy and immunotherapy/biologic therapy.

Chemotherapy in the simplest sense is the treatment of an ailment by chemicals comprising neoplastic diseases, i.e., benigne or maligne tumors. All chemotherapeutic agents cause more or less severe side effects, which make it sometimes even difficult to treat a patient dependent on the general health condition of the patient. There exists a high interest in increasing the efficacy of chemotherapeutic agents and decreasing the side effects at the same time to improve the treatment of neoplastic diseases. Possibilities to reach this goal are for example to increase the sensitivity of the tumor cell against the chemotherapeutic agents or to increase the absorption of the chemotherapeutic agent into the tumor cell.

JP 2005213229 is directed to an antineoplastic agent containing DPA in combination with an immunotherapeutic agent such as an IL-12 production inducing agent.

WO 97/39749 A2 refers to a method for the prevention and treatment of cachexia and anorexia by the use of a nutritional composition comprising fatty acids such as EPA, DPA, and DHA. This composition might also improve the transport of a chemotherapeutic agent into the target tumor cell.

WO 2010/033424 A2 describes an immunonutritional composition comprising amongst others essential fatty acids such as EPA and DHA, which might be administered together with a chemotherapeutic agent.

However, none of these documents disclose EPA, DPA and/or DHA in specific concentrations, which are highly efficient in the prevention and/or treatment of a neoplastic disease.

N-3 PUFAs are of great nutritional importance since the human body is not able to synthesize these n-3 fatty acids itself. It has to form long chain n-3 fatty acids like EPA (C20:5) and DHA (C22:6) from 'short chain' 18 carbon n-3 fatty acids via elongation. Another n-3 PUFA is DPA (C22:5), which is an elongated metabolite of EPA and is an intermediary product between EPA and DHA. Sources of DPA are for example DPA as free fatty acid, or present in phospholipids, or present in triacylglycerol or diacylglycerol, in this example the lipids can be produced synthetically. Other examples of sources of DPA are marine oil, oil from algae, salmon oil, or shark oil. Alternatively, DPA is enzymatically enriched for example in marine oil or algae oil. Many health benefits are described to n-3 PUFAs including cardiovascular, inflammatory, neurogenerative diseases, and cancer.

As the modes of action of the chemotherapeutic agents differ widely, and tumor cells behave very differently depending on the tumor type, the treatment of a neoplastic disease is highly specific. The present invention refers to an unexpected combination of EPA, DPA, and/or DHA and a chemotherapeutic agent and a chemotherapeutic agent, respectively, wherein the concentrations of the active compounds, i.e., EPA, DPA, and/or DHA, and their combination with a chemotherapeutic agent are highly successful for use in preventing and/or treating a neoplastic disease.

Moreover, surprisingly, a chemotherapeutic agent showed higher toxicity on tumor cells and in parallel lower toxicity on healthy, non-tumor-cells, when the chemotherapeutic agent is combined with EPA or DPA and/or high concentration of protein and/or leucine.

### Summary

The present invention relates to a combination of eicosapentaenoic acid (EPA), docosapentaenoic acid (DPA), and/or docosahexanoic acid (DHA), wherein EPA is in an amount of 1 to 1000 mg/100ml, preferably 1 to 700 mg/100ml, DPA is in an amount of > 50 mg/100ml, 50 to 1000 mg/100ml, preferably 65 to 800 mg/100ml, more preferably 80 to 500 mg/100ml and/or DHA is in an amount of 1 to 500 mg/100ml, preferably 1 to 300 mg/100ml and a chemotherapeutic agent, and/or radiotherapy.

In a further embodiment, the present invention refers to a combination of EPA, DPA, and/or DHA, wherein EPA is in an amount of 1 to 1000 mg/100ml, preferably 1 to 700 mg/100ml, DPA is in an amount of > 50 mg/100ml, preferably 50 to 1000 mg/100ml, preferably 65 to 800 mg/100ml, more preferably 80 to 500 mg/100ml, and/or DHA is in an amount of 1 to 500 mg/100ml, preferably 1 to 300 mg/100ml, and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme and a chemotherapeutic agent, and/or radiotherapy.

The chemotherapeutic agent is an alkylating drug, an antimetabolite, an antimytotic cytostatic, a topoisomerase inhibitor, antitumor antibiotic, or any other cytostatic, preferably cisplatin and/or doxorubicin.

In another embodiment the combination of EPA, DPA, and/or DHA, or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme are for use in the treatment of a neoplastic disease, preferably an adenocarcinoma.

An alternative embodiment of the invention is directed to a method for treating a neoplastic disease, comprising administering EPA, DPA, and/or DHA, or a nutritional composition comprising EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, in combination with a chemotherapeutic agent, preferably cisplatin and/or doxorubicin, and/or radiotherapy.
In an additional embodiment, the invention relates to a composition comprising or consisting of DPA in an amount of > 50 mg/100ml, in particular 50 to 1000 mg/100ml, preferably 65 to 800 mg/100ml, more preferably 80 to 500 mg/100ml, or EPA in an amount of 1 to 3000 mg/100ml, 1 to 1000 mg/100ml, more preferably 1 to 700 mg/100 ml, wherein EPA is a depot for DPA and EPA is metabolized into DPA in an organism. The composition is preferably used in the prevention and/or treatment of a neoplastic disease.

The invention relates to a nutritional composition comprising EPA, DPA and/or DHA, and a protein, a carbohydrate, a lipid , an amino acid, and/or a fatty acid, wherein the protein content is preferably > 10en%, or >15en%, or >20en%, and the leucine content is >5%, .>10%, >15%, or >17% of total protein content based on weight.

### Figures

The following figures show specific embodiments of the invention and the present invention is not limited to the following figures.

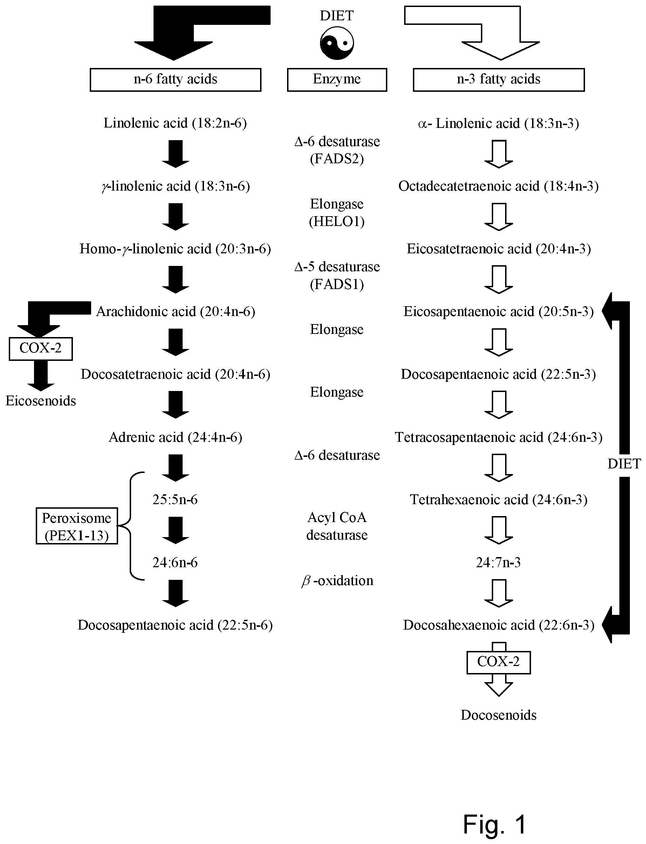

**Fig. 1** shows elongation of polyunsaturated fatty acids.
**Fig. 2** presents an overview of categories of chemotherapeutic.
**Fig. 3** presents pre-incubation of C26 cells with EPA or DPA for 4 days that result in the same ratio of EPA and DPA. Values represent means ± SEM, n=4.
**Fig. 4** shows metabolic activity of C26 cells after 4 days pre-incubation with EPA, DHA or DPA (50 µM) and 24 h incubation with cisplatin (Fig. 4A) or doxorubicin (Fig. 4B). Values represent means ± SEM, n=4.
**Fig. 5** shows skeletal muscle function based on force frequency curves (ex-vivo) in tumor bearing mice after receiving a specific nutritional combination. C = mice receiving control diet B, TB = tumor-bearing mice receiving control diet, TB-SNC = tumor-bearing mice receiving the specific nutritional combination. Data are means ± SEM.
**Fig. 5A** presents the maximal force production, i.e., curves represent the max force production relative to muscle mass of EDL muscle of mice receiving different diets for 20 days after tumor inoculation.
**Fig. 5B** shows the maximal contraction velocity, i.e., curves represent the max contraction velocity relative to muscle mass of EDL muscle of mice receiving different diets for 20 days after tumor inoculation.
**Fig. 5C** shows the maximal relaxation velocity, i.e., curves represent the max relaxation velocity relative to muscle mass of EDL muscle of mice receiving different diets for 20 days after tumor inoculation.

### Detailed description of the invention

The present invention refers to the combination of eicosapentaenoic acid (EPA), docosapentaenoic acid (DPA), and/or docosahexanoic acid (DHA) and a chemotherapeutic agent, or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme and a chemotherapeutic agent. The advantageous effect of n-3 polyunsaturated fatty acids (PUFAs) such as EPA and DHA, e.g., as part of fish oil, for example salmon or shark oil, on health conditions of mammals, in particular humans, is often discussed. As DPA is an intermediary product between EPA and DHA, which is hardly available in pure form and forms only a minor part for example in fish oil, the effect of DPA is less investigated. The present invention shows that a combination of these n-3 PUFAs, in particular of DPA, and a chemotherapeutic agent significantly increases the cytotoxic effect of the chemotherapeutic agent, which is measurable in the metabolic activity. Thus, EPA, DPA, and/or DHA increase the sensitivity of the cells against the chemotherapeutic agent, wherein DPA is the most efficient n-3 PUFA. In addition, a chemotherapeutic agent has a much higher toxicity on a tumor cell, than on a healty, non-tumor cell, when a nutritional or pharmaceutical composition comprising EPA, and/or DPA and a high protein and/or leucin concentration.

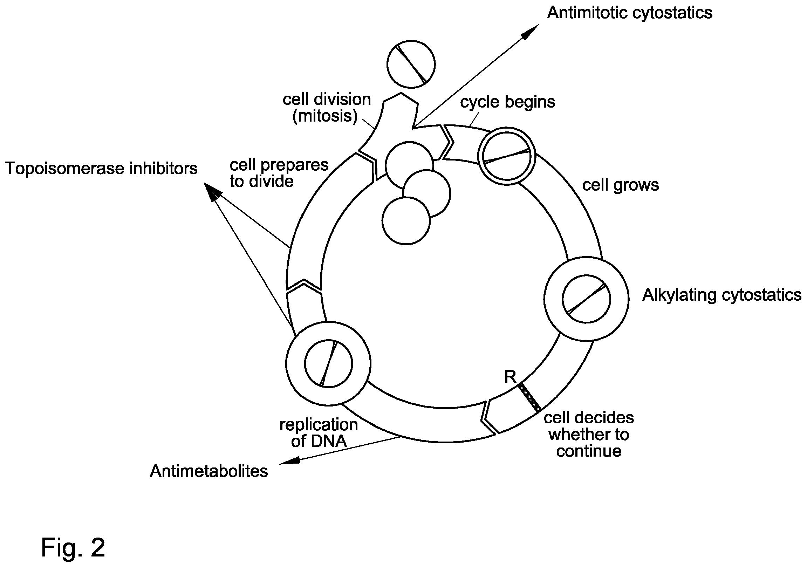

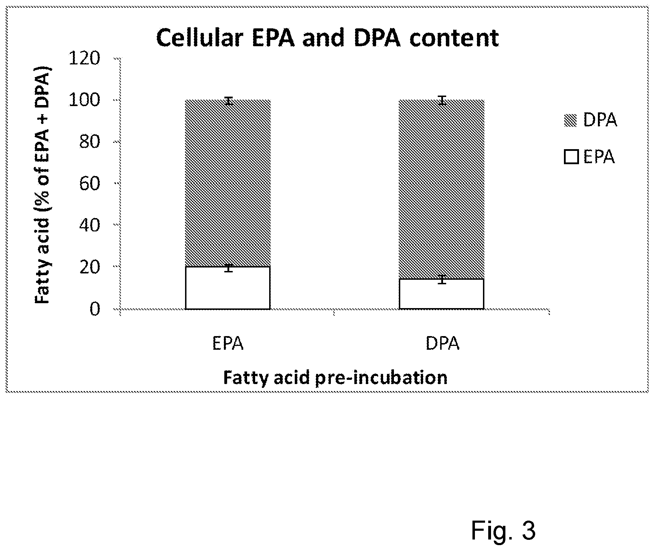

EPA, which is metabolized to DPA (see Fig. 2), has a double effect on the cells in this context. One is the directly sensitising effect on the tumor cells, another is the "depot effect", which is based on the metabolizing of EPA to the highly effective DPA (see example 1 and Fig. 3). The selection and combination of EPA, DPA, and/or DHA, their concentrations as well as the selection of the chemotherapeutic agent, provide a highly specific combination, which leads to a very selective and efficient use in treating a neoplastic disease.

A neoplastic disease in the present invention is any benign or malingn tumor. A benign tumor lacks the ability to metastasize, and the term "benign" implies a mild and nonprogressive disease. Indeed, many kinds of benign tumors are harmless to human health. However, some neoplasms defined as 'benign tumors' may still produce negative health effects. Examples of this include tumors which produce a "mass effect" (compression of vital organs such as blood vessels), or tumors of endocrine tissues, which may overproduce certain hormones. Examples include thyroid adenomas, adrenocortical adenomas, and pituitary adenomas. Benign tumors typically are surrounded by an outer surface (fibrous sheath) that inhibits their ability to behave in a malignant manner. Nonetheless, many types of benign tumors have the potential to become malignant and some types, such as teratoma, are notorious for this.

A malignant tumor is not self-limited in its growth, different than a benign tumor. It is capable of invading into adjacent tissues, and may be capable of spreading to distant tissues (metastasizing). Malignant tumor in the present invention is synonymous with cancer.

The combinations of the present invention are used in treating neoplastic diseases for example pancreatic cancer, melanoma, brain tumor, bladder cancer, breast cancer, bladder cancer, ovary cancer, prostate cancer, colon cancer, gastric cancer, endometrial cancer, sarcoma, blood born tumor, leukemia, blastoma, glioma, mesothelioma, neuroblastoma, kidney cancer, liver cancer, lung cancer, melanoma, preferably adenocarcinoma. The method for treating a neoplastic disease is likewise preferably directed to the treatment of these types of cancer, in particular adenocarcinoma.

Adenocarcinoma is a cancer of an epithelium that originates in glandular tissue. Epithelial tissue includes, but is not limited to, the surface layer of skin, glands and a variety of other tissue that lines the cavities and organs of the body. Epithelium can be derived embryologically from ectoderm, endoderm or mesoderm. To be classified as adenocarcinoma, the cells do not necessarily need to be part of a gland, as long as they have excretory properties. This form of carcinoma occurs for example in mammals such as humans, dogs, cats, horses etc.

The combinations of the present invention comprise or consist of EPA, DPA, and/or DHA, or EPA, DPA, and/or DHA, and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, and a chemotherapeutic agent. Several types of chemotherapeutics exist, which differ in their mode of action for example to attack and eliminate a cancer cell, and which will be presented in the following. Fig. 2 shows the different effects of these chemotherapeutics agents.

One type is alkylating agents that affect all phases of the cell cycle. The alkylating cytostatics can be diveded in three different classes due to their different mode of action, however, all of these alkylating cytostatics result in alkyl groups binding to negatively charged groups of the DNA (O₂, N₂, P and S atoms). The alkylation leads to the disruption of the DNA function and the cell death. Examples of alkylating agents are cisplatin, oxaliplatin, cyclophosphamid, ifosfamid, trofosfamid, melphalan, chlorambucil, estramustin, busulfan, treosulfan, carmustin, lomustin, nimustin, streptozocin, procarbazin, dacarbazin, temozolomid, and thiotepa.

Further types of chemotherapeutic agents are antimetabolites that interfere with the formation of biomolecules in the cell, and are mainly active during the S phase of the cell cycle. Antimetabolites are structurally similar to metabolites, but cannot be used, e.g., metabolized, by the organism in a productive manner. Thus, antimetabolites mimic natural metabolites and are processed in the cell analogous to the natural metabolite. The antimetabolites block the vital functions of the cell, the cell is unable to grow and will die. 5-fluorouracil, methotrexate, azacitidin, capecitabin, doxifluridin, cytarabin, gemcitabin, 6-thioguanin, pentostatin, azathioprin, 6-mercaptopurin, fludarabin, and cladribin are examples for antimetabolites.

Antimytotic cytostatics represent another type of chemotherapeutic agents, which effect the M phase of the cell cycle. Microtubules, which play a key role in the M phase, are the main target for antimytotic cytostatics. The antimytotic cytostatics block the microtubules and thus, the cell cycle. Examples of antimytotic cytostatics are vinorelbin, vincristin, vinblastin, and vindesin.

Topoisomerase inhibitors represent a further type of chemotherapeutic agents. DNA topoisomerases are responsible for the topological state of the DNA in the cell, and the enzymes catalyze a three step process which comprises 1) cleavage of one or both DNA strands depending on the type of topoisomerase (topoisomerase I cleaves one, topoisomerase II both strands), 2) passage of a segment of DNA through this breach, and 3) resealing of the DNA breach. The inhibitor of the topoisomerases blocks the ligation step of the cell cycle, generating single and double stranded breaks that harm the integrity of the genome. Introduction of these breaks subsequently lead to apoptosis and cell death. Examples of topoisomerase inhibitors are doxorubicin, camptothecin, topotecan, irinotecan, etoposid, and teniposid.

Another type of chemotherapeutic agents is antitumor antibiotics, which are chemotherapeutic agents made mostly from natural products e.g., produced by species of the soil fungus Streptomyces. These drugs act during multiple phases of the cell cycle and are considered cell-cycle specific. Examples of antitumor antibiotics are tamoxifen, 5-fluoro-5'-deoxyuridine, belomycin, actinomycin D, and mitomycin.

Remaining cytostatics represent an alternative group of chemotherapeutic agents, which are based on very different mode of actions. L-asparaginase for example is a L-asparagine degrading enzyme, which attacks specifically leukemic cells that are not able to produce L-asparagine theirselves. Hydroxycarbamide for example inhibits the enzyme ribonucleotide reductase, and thus, DNA synthesis, which is particulary effective on leukemic cells. Mitotan for example affects tumor cell metabolism, in particular in renal cells, amatoxine inhibits RNA polymerase and thus, RNA transcription and inconsequence protein synthesis, and altretamin inhibits DNA- and RNA synthesis in the cell nucleus.

Radiotherapy like a chemotherapeutic agent is used in treating malign and benign tumors. Therefore, the present invention refers in an alternative embodiment to a combination of EPA, DPA, and/or DHA, or EPA, DPA, and/or DHA, and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, and radiotherapy.

Beside EPA, DPA, and/or DHA, the combination of the present invention comprises in a preferred embodiment a protein, e.g., animal protein such as whey protein or casein, or a vegetable protein such a soy protein or wheat protein, carbohydrate, e.g., sugars such as glucose, fructose, galactose, lactose, maltose, sucrose, or trehalose, polysaccharides such as starch, or organic acids, fat, e.g., saturates, monounsatirates, polyunsaturates, or cholesterol, amino acid, e.g., the L-form of alanine, arginine, aspartic acid, asparagine, cyst(e)ine, histidine, isoleucine, leucine, lysine, methionine, phenylalanine, proline, serine, threonine, tryptophan, tyrosine, or valine, and/or a fatty acid, preferably butyric acid, caproic acid, caprylic acid, capric acid, lauric acid, myristic acid, palmitic acid, palmitate, palmitoleic acid, stearic acid, oleic acid, linoleic acid, alpha linolenic acid, gamma linolenic acid, arachidic acid, eicosaenioc acid, dihomo gamma-linolenic acid, arachidonic acid, behenic acid, erucic acid, lignoceric acid, and nervoic acid. Optionally the nutritional composition comprises a dietary fibre, e.g., soluble or unsoluble, a vitamin, e.g., vitamin A, D3, E, K, B6, B12, C, thiamine, riboflavin, niacin, pantothenic acid, folic acid, or biotin, a mineral, e.g., sodium, potassium, chloride, phosphorus, magnesium, iron, zinc, copper, manganese, fluoride, molybdenum, selenium, chromium, or iodine, a trace element, β-carotenoid, e.g., α-, β-, or γ-carotene, lycopene, lutein, or zeaxanthin, a flavonoid, e.g., hesperidin or quercetin, a nucleotide, e.g., cytidine-5-monophosphate, uridine-5'-monophosphate, adenosine-5-monophosphate, guanosine-5-monophosphate, or inosine-5'-monophosphate, L-carnitin, choline, inositol, taurine, creatine, and/or a co-enzyme, e.g., co-enzyme Q10.

In a preferred embodiment, a pharmaceutical or nutritional composition of the present invention comprises or consist of EPA, DPA, and/or DHA, or EPA, DPA, and/or DHA, and a protein, a carbohydrate, a Lipid (fat) an amino acid, and/or a fatty acid.

In a preferred embodiment the combination, preferably the nutritional or pharmaceutical composition comprises EPA, DPA, and/or DHA, whey protein, casein and nitrogen, glucose, galactose, lactose, maltose, sucrose, and trehalose, organic acid, vegetable and animal fat, preferably of milk, butyric acid, caproic acid, caprylic acid, capric acid, lauric acid, myristic acid, palmitic acid, palmitate, palmitoleic acid, stearic acid, oleic acid, linoleic acid, alpha linolenic acid, arachidic acid, eicosaenioc acid, arachidonic acid, behenic acid, and erucic acid, soluble dietary fibres, sodium, potassium, chloride, phosphorus, magnesium, iron, zinc, copper, manganese, fluoride, molybdenum, selenium, chromium, and iodine, vitamin A, D3, E, K, B6, B12, C, thiamine, riboflavin, niacin, pantothenic acid, folic acid, and biotin, α-, β-, or γ-carotene, lycopene, lutein, and zeaxanthin, L-carnitine, choline, and taurine, the L-form of alanine, arginine, aspartic acid, asparagine, cyst(e)ine, histidine, isoleucine, leucine, lysine, methionine, phenylalanine, proline, serine, threonine, tryptophan, tyrosine, and valine, and optionally water.

In a further preferred embodiment, the combination, preferably the nutritional or pharmaceutical composition, comprises one or more chemotherapeutic agents such as cisplatin, oxaliplatin, cyclophosphamid, ifosfamid, trofosfamid, melphalan, chlorambucil, estramustin, busulfan, treosulfan, carmustin, lomustin, nimustin, streptozocin, procarbazin, dacarbazin, temozolomid, thiotepa, 5-fluorouracil, methotrexate, azacitidin, capecitabin, doxifluridin, cytarabin, gemcitabin, 6-thioguanin, pentostatin, azathioprin, 6-mercaptopurin, fludarabin, cladribin, vinorelbin, vincristin, vinblastin, vindesin, doxorubicin, camptothecin, topotecan, irinotecan, etoposid, teniposid, tamoxifen, 5-fluoro-5'-deoxyuridine, belomycin, actinomycin D, mitomycin, L-asparaginase, hydroxycarbamide, mitotan, amatoxine, and/or altretamin, preferably cisplatin and/or doxorubicin. A combination of chemotherapeutic agents comprises for example 5-fluorouracil, oxaliplatin, and folic acid (Folfox 4).

In a preferred embodiment, the chemotherapeutic agent is administered for 1h, 3h, 6h, 8h, 10h, 12h, 1d, 2d, 3d, 4d, 5d, 6d, or 7d, preferably prior to or subsequent to the administration of EPA, DPA, and/or DHA, or EPA, DPA, and/or DHA and a protein, a carbohydrate, a lipid (fat,) an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, more preferably in parallel.

The combination of EPA, DPA, and/or DHA, or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, and one or more chemotherapeuic agents, comprise EPA in an amount of 1 to 3000 mg/100 ml, 1 to 1000 mg/100ml, preferably 1 to 700 mg/100ml, DPA is in an amount of 1 or 50 to 1000 mg/100ml, preferably 1 or 50 to 500 mg/100ml, more preferably 3, 30 or 50 to 300 mg/100ml, alternatively > 50 mg/100ml, e.g., 50 to 100 mg/100 ml, preferably 65 to 800 mg/100ml, more preferably 80 to 500 and/or DHA is in an amount of 1 to 500 mg/100ml, preferably 1 to 300 mg/100ml. The 100 ml refer to the volume of EPA, DPA, and/or DHA, or of EPA, DPA, and/or DHA and a protein, a carbohydrate, a lipid (fat), an amino acid, a fatty acid, a dietary fibre, a vitamin, a mineral, a trace element, B-carotenoid, a flavonoid, a nucleotide, L-carnitin, choline, inositol, taurine, creatine, and/or a co-enzyme excluding the chemotherapeutic agent

EPA, DPA, and/or DHA, or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered to a tumor cell prior to, in parallel to, or subsequent to the administration of a chemotherapeutic agent, and/or radiotherapy.

In an embodiment, EPA, DPA, and/or DHA, or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered 1h, 2h, 3h, 4h, 5h, 6h, 7h, 8h, 9h, 10h, 11h, 12h, or 1d, 2d, 3d, 4d, 5d, 6d, or 7d to a tumor cell prior to or subsequent to the administration of a chemotherapeutic agent, and/or radiotherapy.

In another embodiment, EPA, DPA, and/or DHA or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered 1 to 3x daily, 1 to 3x weekly, or 1 to 3x monthly.

EPA, DPA, and/or DHA, or EPA, DPA, and/or DHA and a protein, a carbohydrate, alipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzymeis administered in form of a powder, a tablet, a capsule, or a fluid. Preferably a pharmaceutical or a nutritional composition is administered in liquid form.

The combination of EPA, DPA, and/or DHA, or EPA, DPA, and/or DHA and a protein, a carbohydrate, a lipid (fat), an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, and one or more chemotherapeutic agents, and/or radiotherapy is for use in treating a neoplastic disease, alternatively, for use in a method of treating a neoplastic disease or for use in an *in vitro* method. In particular, EPA, DPA and/or DHA, or EPA, DPA and/or DHA and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme, enhance the effect of a chemotherapeutic agent and/or radiotherapy.

In another embodiment, the invention refers to a composition comprising or consisting of DPA in an amount of 1 or 50 to 1000 mg/100ml, preferably 1 or 50 to 500 mg/100ml, more preferably 3, 30 or 50 to 300 mg/100ml, alternatively > 50 mg/100ml, e.g., 50 to 100 mg/100 ml, preferably 65 to 800 mg/100ml, more preferably 80 to 500 mg/100ml or EPA in an amount of 1 to 3000 mg/100ml, preferably 1 to 1000 mg/100ml, more preferably 100 to 700 mg/100 ml, wherein EPA is a depot for DPA and EPA is metabolized into DPA in an organism. The composition is preferably used in the prevention and/or treatment of a neoplastic disease.

The invention further comprises a method for preventing and/or treating a neoplastic disease comprising administering DPA in an amount of 1 or 50 to 1000 mg/100ml, preferably 1 or 50 to 500 mg/100ml, more preferably 3, 30 or 50 to 300 mg/100ml, alternatively > 50 mg/100ml, e.g., 50 to 100 mg/100 ml, preferably 65 to 800 mg/100ml, more preferably 80 to 500 mg/100ml or EPA in an amount of 1 to 3000 mg/100ml, preferably 1 to 1000 mg/100ml, more preferably 100 to 700 mg/100 ml, wherein EPA is a depot for DPA and is metabolized into DPA in an organism.

In a further embodiment, the invention relates to the combination or the nutritional composition comprising EPA, DPA and/or DHA, and a protein, a carbohydrate, a lipid (fat), an amino acid, and/or a fatty acid, wherein the protein content is preferably > 10en%, e.g., 10 to 100en%, 10 to 80en%, 10 to 50en%, or 10 to 30en% , or >15en%, e.g., 15 to 100en%, 15 to 80en%, 15 to 50en%, or 15 to 30en%, or >20en%, e.g., 20 to 100en%, 20 to 80en%, 20 to 50en%, or 20 to 30en%, and the leucine content is >5%, >10%, >15%, or >17% of total protein content, preferably 5 to 100%, 5 to 80%, 5 to 50%, 5 to 30%, 10 to 100%, 10 to 80%, 10 to 50%, 10 to 30%, 15 to 100%, 15 to 80%, 15 to 50%, 15 to 30%, or 17 to 100%, 17 to 80%, 17 to 50%, or 17 to 30% of total protein content based on weight. Preferably, the protein comprises at least one protein from a protein source selected from the group consisting of casein, caseinate, soy and wheat. In particular, the protein comprises at least 15wt%, preferably at least 25wt% of whey based on the total protein content.

Alternatively, the combination or the nutritional composition of the present invention contains the lipid in a content of >10% of the sum of EPA and DPA based on weight and DPA in a content of >5%, preferably >10%, more preferably >20% of total (EPA and DPA).

In a further alternative, the lipid content contains or the fatty acid is selected from the group consisting of butyric acid, caproic acid, caprylic acid, capric acid, lauric acid, myristic acid, palmitic acid, palmitate, palmitoleic acid, stearic acid, oleic acid, linoleic acid, alpha linolenic acid, gamma linolenic acid, arachidic acid, eicosaenioc acid, dihomo gamma-linolenic acid, arachidonic acid, behenic acid, erucic acid, lignoceric acid, and nervoic acid.

The following combinations of features are meant to be part of the present application.
1. Combination comprising eicosapentaenoic acid (EPA), or docosapentaenoic acid (DPA), wherein EPA is in an amount of 1 to 1000 mg/100ml, preferably 1 to 700 mg/100ml, DPA is in an amount of more than 50 mg/100ml, and a chemotherapeutic agent selected from the group consisting of an alkylating drug, an antimetabolite, an antimytotic cytostatic, a topoisomerase inhibitor, antitumor antibiotic, and any other cytostatic, and/or radiotherapy.
2. Combination according to POINT 1, wherein DPA is in an amount of 50 to 1000 mg/100ml, preferably 65 to 800 mg/100ml, more preferably 80 to 500 mg/100ml.
3. Combination according to POINT 1 or 2, further comprising a protein, a carbohydrate, a lipid, an amino acid, preferably leucine, and/or a fatty acid.
4. Combination according to POINT 3, wherein the lipid is in a content of more than 10% of the sum of EPA and DPA based on weight and DPA is in a content of more than 5%, preferably more than 10%, more preferably more than 20% of total (EPA and DPA).
5. Combination according to POINT 3 or 4, wherein the lipid content contains or the fatty acid is selected from the group consisting of butyric acid, caproic acid, caprylic acid, capric acid, lauric acid, myristic acid, palmitic acid, palmitate, palmitoleic acid, stearic acid, oleic acid, linoleic acid, alpha linolenic acid, gamma linolenic acid, arachidic acid, eicosaenioc acid, dihomo gamma-linolenic acid, arachidonic acid, behenic acid, erucic acid, lignoceric acid, and nervoic acid.
6. Combination according to any of POINTS 3 to 5, wherein the protein content is higher than 10 en%, or higher than 15 en%, or higher than 20 en%, and the leucine content is higher than 5%, or higher than 10%, or higher than 15%, or higher than 17% of the total protein content based on weight.
7. Combination according to any of POINTS 3 to 6, wherein the protein comprises at least one protein from a protein source selected from the group consisting of casein, caseinate, soy and wheat.
8. Combination according to any of POINTS 3 to 7, wherein the protein comprises at least 15wt%, preferably at least 25wt% of whey based on the total protein content.
9. Combination according to any of POINTS 1 to 8, further comprising a dietary fibre, a vitamin, a mineral, a trace element, β-carotenoid, a flavonoid, a nucleotide, L-carnitin, choline, inositol, taurine, creatine, and/or a co-enzyme.
10. Combination according to any of POINTS 1 to 9, wherein the alkylating drug is selected from the group consisting of cisplatin, oxaliplatin, cyclophosphamid, ifosfamid, trofosfamid, melphalan, chlorambucil, estramustin, busulfan, treosulfan, carmustin, lomustin, nimustin, streptozocin, procarbazin, dacarbazin, temozolomid, and thiotepa; the antimetabolite is selected from the group consisting of 5-fluorouracil, methotrexate, azacitidin, capecitabin, doxifluridin, cytarabin, gemcitabin, 6-thioguanin, pentostatin, azathioprin, 6-mercaptopurin, fludarabin, and cladribin; the antimytotic cytostatic is selected from the group consisting of vinorelbin, vincristin, vinblastin, and vindesin; the topoisomerase inhibitor is selected from the group consisting of doxorubicin, camptothecin, topotecan, irinotecan, etoposid, and teniposid; the antitumor antibiotic is selected from the group consisting of tamoxifen, 5-fluoro-5'-deoxyuridine, belomycin, actinomycin D, and mitomycin; and/or the cytostatic is selected from the group consisting of L-asparaginase, hydroxycarbamide, mitotan, amatoxine, and altretamin.
11. Combination according to any of POINTS 1 to 10, wherein EPA or DPA, or EPA or DPA, and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is a pharmaceutical or a nutritional composition.
12. Combination according to any of POINTS 1 to 11 for use in treating a neoplastic disease.
13. Combination according to POINT 12, wherein the neoplastic disease is a benign or malign tumor.
14. Combination according to POINT 12 or 13, wherein the neoplastic disease is selected from the group consisting of pancreatic cancer, melanoma, brain tumor, bladder cancer, breast cancer, bladder cancer, ovary cancer, prostate cancer, colon cancer, gastric cancer, endometrial cancer, sarcoma, blood born tumor, leukemia, blastoma, glioma, mesothelioma, neuroblastoma, kidney cancer, liver cancer, lung cancer, and melanoma.
15. Combination according to any of POINTS 11 to 14, wherein EPA or DPA, or EPA or DPA and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered prior to the chemotherapeutic agent, in parallel with the chemotherapeutic agent, or subsequent to the chemotherapeutic agent, and/or radiotherapy.
16. Combination according to any of POINTS 11 to 15, wherein EPA or DPA, , or EPA or DPA and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered 1 to 7 days, preferably 1 to 5, more preferably 1 to 3 days before and/or after the chemotherapeutic agent.
17. Combination according to any of POINTS 11 to 16, wherein EPA or DPA, or EPA or DPA and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered daily, weekly, or monthly.
18. Combination according to any of POINTS 11 to 17, wherein EPA or DPA, or EPA or DPA and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered in form of a powder, a tablet, a capsule, or a fluid.
19. Method for treating a neoplastic disease, comprising administering EPA or DPA, or EPA or DPA and a protein, a carbohydrate, a fat, an amino acid, and/or a fatty acid, wherein EPA is administered in an amount of 1 to 1000 mg/100ml, preferably 1 to 700 mg/100ml, DPA is administered in the amount of more than 50 mg/100 ml in combination with a chemotherapeutic agent selected from the group consisting of an alkylating drug, an antimetabolite, an antimytotic cytostatic, a topoisomerase inhibitor, antitumor antibiotic, and any other cytostatic, and/or radiotherapy to a subject.
20. Method according to POINT 19, wherein DPA is administered in an amount of 50 to 1000 mg/100ml, preferably 65 to 800 mg/100ml, more preferably 80 to 500 mg/100ml.
21. Method according to POINT 19 or 20, wherein the alkylating drug is selected from the group consisting of cisplatin, oxaliplatin, cyclophosphamid, ifosfamid, trofosfamid, melphalan, chlorambucil, estramustin, busulfan, treosulfan, carmustin, lomustin, nimustin, streptozocin, procarbazin, dacarbazin, temozolomid, and thiotepa; the antimetabolite is selected from the group consisting of 5-fluorouracil, methotrexate, azacitidin, capecitabin, doxifluridin, cytarabin, gemcitabin, 6-thioguanin, pentostatin, azathioprin, 6-mercaptopurin, fludarabin, and cladribin; the antimytotic cytostatic is selected from the group consisting of vinorelbin, vincristin, vinblastin, and vindesin; the topoisomerase inhibitor is selected from the group consisting of doxorubicin, camptothecin, topotecan, irinotecan, etoposid, and teniposid; the antitumor antibiotic is selected from the group consisting of tamoxifen, 5-fluoro-5'-deoxyuridine, belomycin, actinomycin D, and mitomycin; and/or the cytostatic is selected from the group consisting of L-asparaginase, hydroxycarbamid, mitotan, amatoxine, and altretamin.
22. Method according to any of POINTS 19 to 21, wherein the subject is a mammalian, preferably a human being.
23. Method according to any of POINTS 19 to 22, wherein the neoplastic disease is selected from the group consisting of pancreatic cancer, melanoma, brain tumor, bladder cancer, breast cancer, bladder cancer, ovary cancer, prostate cancer, colon cancer, gastric cancer, endometrial cancer, sarcoma, blood born tumor, leukemia, blastoma, glioma, mesothelioma, neuroblastoma, kidney cancer, liver cancer, lung cancer, melanoma.
24. Method according to any of POINTS 19 to 23, wherein EPA or DPA, or EPA or DPA and a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme is administered in form of a powder, a tablet, a capsule, or a fluid.
25. A nutritional composition comprising or consisting of DPA in an amount of more than 50 mg/100ml.
26. Composition according to POINT 25 comprising or consisting of DPA in an amount of 50 to 1000 mg/100ml, preferably 65 to 800 mg/100ml, more preferably 80 to 500 mg/100ml.
27. A composition comprising or consisting of EPA in an amount of 1 to 3000 mg/100ml, preferably 1 to 1000 mg/100ml, more preferably 1 to 700 mg/100 ml, wherein EPA is a depot for DPA and EPA is metabolized into DPA in an organism.
28. Composition according to any of POINTS 25 to 27 further comprising a protein, a carbohydrate, a fat, an amino acid, a fatty acid, a dietary fibre, a vitamin, a mineral, a trace element, β-carotenoid, a flavonoid, a nucleotide, L-carnitin, choline, inositol, taurine, creatine, and/or a co-enzyme.
29. Composition according to any of POINTS 25 or 28 for use in preventing and/or treating a neoplastic disease.
30. Composition according to any of POINTS 25 to 29 in which the composition is presented as a powder, a tablet, a capsule, or a fluid.
31. Composition according to POINT 30, wherein the composition is a powder for the preparation of a ready to use solution by adding a liquid.
32. Method for preventing and/or treating a neoplastic disease comprising administering DPA in an amount of more than 50 mg/100ml.
33. Method according to POINT 32 comprising administering DPA in an amount of 50 to 1000 mg/100ml, preferably 65 to 800 mg/100ml, more preferably 80 to 500 mg/100ml.
34. Method for preventing and/or treating a neoplastic disease comprising administering EPA in an amount of 1 to 3000 mg/100ml, preferably 1 to 1000 mg/100ml, more preferably 1 to 700 mg/100 ml, wherein EPA is a depot for DPA and is metabolized into DPA in an organism.

The present invention is shown in more detail in the following examples, wherein the invention is not limited to these examples.

### Example 1

### Total fatty acid analysis

Murine C26 adenocarcinoma cells are routinely cultured twice a week, and cell cultures are studied by a binocular to determine confluency. Culture medium is removed by vacuum suction and cells are washed once with 10 ml of pre-warmed PBS. PBS is removed and 2 ml Trypsin-EDTA is added to the culture flask. After a short incubation period in the incubator, detachment of the cells is determined by studying the cells with a binocular. Cells are taken up in 8 ml culture medium and well resuspended. Depending on the confluency of the cell culture, cells are passaged in a new culture flask in a ratio of 1:3 or 1:5. In addition, 25 ml of fresh culture medium is added to the flask.

To determine total phospholipid fatty acids, murine C26 cells were cultured in 6 wells plates. After the incubation period of 4 days with 50 µM EPA, DPA, or DHA, cells were washed with pre-warmed PBS and trypsinized.
After detachment cells were resuspended in 1 ml culture medium and converted in 15 ml
tubes. Cells were centrifuged for 5 minutes at 1500 rpm, supernatant was removed and cells
were lysed by the addition of 500µl cold sterile demineralized water and vortexed. Phospholipid fatty acid content was determined by gas chromatography.

Samples of the stock solutions and the culture medium were also subjected to analysis in order determine the purity of the added fatty acids. Control cells were very high in oleic acid (C18:1 n-9), after pre-incubation with n-3 fatty acids a large fraction of oleic acid was replaced by the added n-3 fatty acid. Pre-incubation with EPA resulted in a small increase in EPA phospholipid content (8.1%) of the cells, but a very high increase in DPA phospholipid content (32.6%). On the other hand, DPA pre-incubation also resulted in an increase in EPA phospholipid content (6.6%) comparable to EPA pre-incubation and a similar increase in DPA phospholipid content (40.8%). This suggests that it makes no difference whether the cells were pre-incubated with EPA or DPA: both fatty acids resulted in the same patern of fatty acid incorporation (see Fig. 3). This phenomenon is due to the intracellular elongation process that converted EPA into DPA, but also retroconversion of DPA into EPA occured. It is possible that there is an optimal ratio of EPA and DPA present in the cells. Pre-incubation of the cells with DHA resulted in an increase in DHA phospholipid content (28%) of the cells.

### Example 2

### Metabolic activity of EPA, DHA or DPA

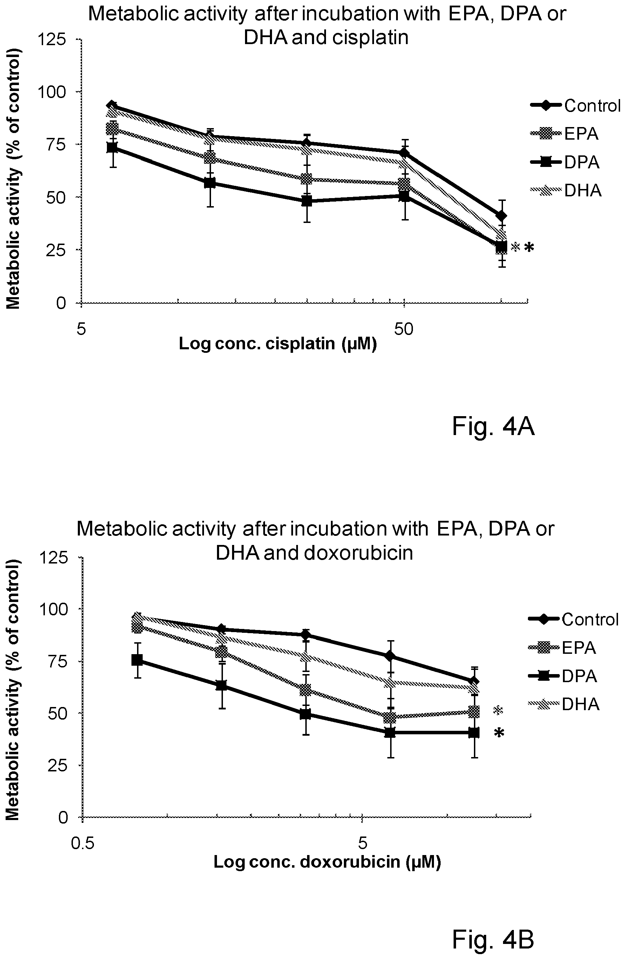

Prior to chemotherapy incubations, C26 cells were pre-incubated for 4 days with EPA, DHA
or DPA in a final concentration of 50 µM. To avoid direct interaction between fatty acids
and chemotherapeutics, the medium containing fatty acids was removed before the addition
of chemotherapy. The cells were incubated for 24 hr with the chemotherapeutics cisplatin and
doxorubicin, after incubation the metabolic activity was measured. Fig. 4 shows the results of the metabolic activity of chemotherapy treated cells that were pre-incubated with EPA, DHA or DPA. Addition of EPA or DPA resulted in a significant lower metabolic activity after cisplatin treatment (p< 0.0001) as well as doxorubicin treated cells (p< 0.0001). DHA showed no significant changes in metabolic activity compared to control.

### Example 3

### Skeletal muscle function

Male CD2F1 mice at 5-6 weeks of age, (BALB/c x DBA/2, Harlan / Charles River the Netherlands) were individually housed in a climate-controlled room (12:12 dark-light cycle with a constant room temperature of 21 ± 1 °C). After acclimatization for one week mice were divided into weight-matched groups: (1) control receiving control chow (C), (2) tumor-bearing receiving control chow (TB), and (3) tumor-bearing receiving a specific nutritional combination (TB-SNC) diet as previously described containing high protein, leucine and fish oil as active ingredients, moreover a specific oligosaccharide mixture was added [1]. All experimental procedures were approved by the Animal Ethical Committee (DEC consult, Bilthoven, The Netherlands) and complied with the principles of good laboratory animal care.

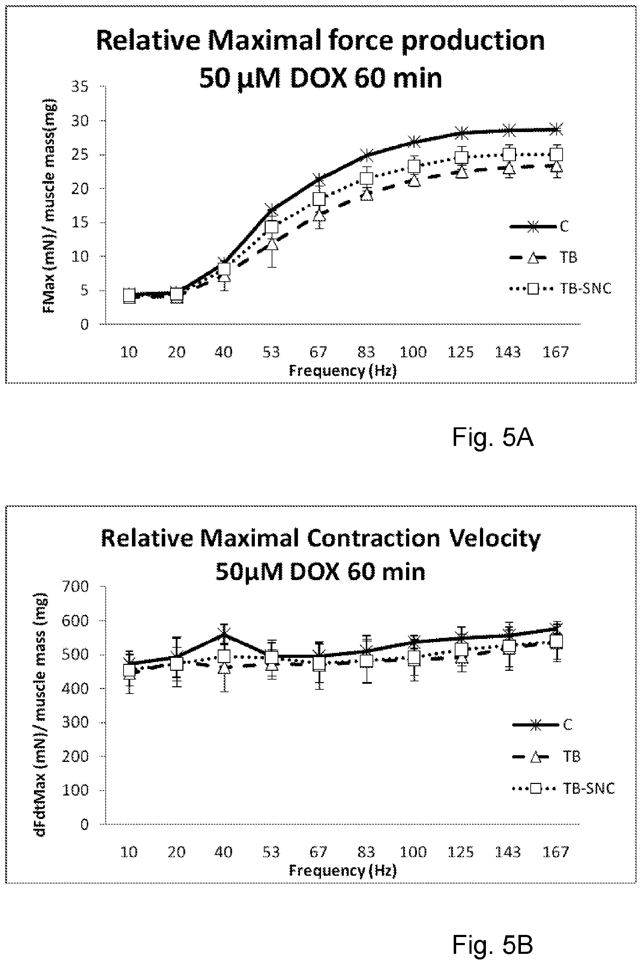

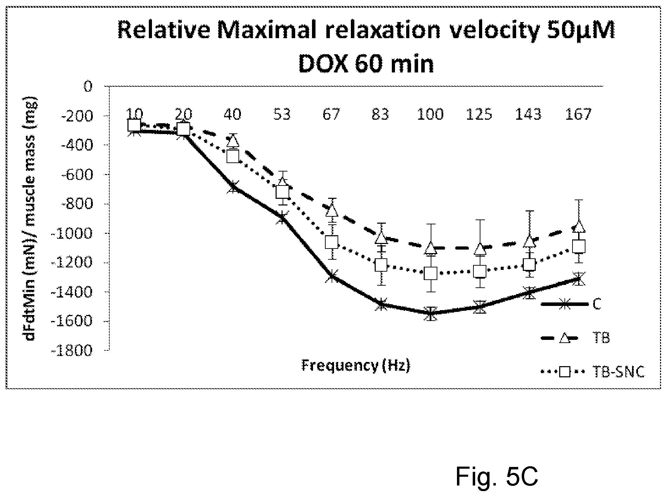

Contractile characteristics of the musculus extensor digitorum longus (EDL) from male CD2F1 mice, 7-9 weeks old, were assessed *ex vivo* as described previously [2]. Muscles were incubated (60 min) with a doxorubicin (DOX) concentration of 50 µM. *Ex vivo* toxicity on non-cancer muscle tissue was measured as reported before [1, 3]. Results are shown in Fig. 5A to Fig. 5C.

### Literature list

1. van Norren, K., et al., Dietary supplementation with a specific combination of high protein, leucine, and fish oil improves muscle function and daily activity in tumour-bearing cachectic mice. Br J Cancer., 2009. 100(5): p. 713-22.
2. Gorselink, M., et al., Mass-dependent decline of skeletal muscle function in cancer cachexia. Muscle Nerve., 2006. 33(5): p. 691-3.
3. van Norren, K., et al., Direct effects of doxorubicin on skeletal muscle contribute to fatigue. Br J Cancer., 2009. 100(2): p. 311-4. Epub 2009 Jan 13.

## Claims

1. EPA and/or DPA for use in reducing side-effects of a chemotherapeutic agent for use in treatment of a neoplastic disease, said chemotherapeutic agent being selected from the group consisting of an alkylating drug, an antimetabolite, an antimytotic cytostatic, a topoisomerase inhibitor, antitumor antibiotic, and any other cytostatic, and/or radiotherapy, wherein the EPA and/or DPA are part of a nutritional composition having a protein content of more than 10 en %, and a leucine content of more than 5 % of total protein content based on weight.

2. EPA and/or DPA for use according to claim 1, wherein the EPA and/or DPA is further for use for enhancing the effect of the chemotherapeutic agent, whereby the chemotherapeutic agent shows higher toxicity on tumor cells and in parallel lower toxicity on healthy non-tumor cells.

3. EPA and/or DPA for use according to claim 1 or 2; wherein EPA is administered to a subject in an amount of 1 to 4000 mg per day, preferably 3 to 3000 mg per day, more preferably 2 to 2000 mg per day.

4. EPA and/or DPA for use according to any of the claims 1-3, wherein DPA is administered to a subject in an amount of more than 50 mg per day, preferably more than 100 mg per day, preferably more than 150 mg per day, more preferably 200 to 4000 mg/day, even more preferably 250 to 3200 mg/day, or even more preferably 300 to 2000 mg/day.

5. A composition, comprising EPA and/or DPA for use according to claim 1 or 2; wherein EPA is in an amount of 1 to 1000 mg/100ml, preferably 1 to 700 mg/100ml, and/or DPA is in an amount of more than 50 mg/100ml.

6. The composition, comprising EPA and/or DPA for use according to claim 5, wherein DPA is in an amount of 50 to 1000 mg/100ml, preferably 65 to 800 mg/100ml, more preferably 80 to 500 mg/100ml.

7. The composition, comprising EPA and/or DPA for use according to claim 5 or 6, further comprising a protein, a carbohydrate, a lipid, an amino acid, preferably leucine, and/or a fatty acid.

8. The composition, comprising EPA and/or DPA for use according to claim 7, wherein the additional lipid is in a content of more than 10% of the sum of EPA and DPA based on weight and DPA is in a content of more than 5%, preferably more than 10%, more preferably more than 20% of total EPA and DPA.

9. The composition, comprising EPA and/or DPA for use according to claim 7 or 8, wherein the additional lipid or additional fatty acid is selected from the group consisting of butyric acid, caproic acid, caprylic acid, capric acid, lauric acid, myristic acid, palmitic acid, palmitate, palmitoleic acid, stearic acid, oleic acid, linoleic acid, alpha linolenic acid, gamma linolenic acid, arachidic acid, eicosaenioc acid, dihomo gamma-linolenic acid, arachidonic acid, behenic acid, erucic acid, lignoceric acid, and nervoic acid.

10. The composition, comprising EPA and/or DPA for use according to any of claims 7 to 9, wherein the protein content is higher than 10 en%, or higher than 15 en%, or higher than 20 en%, and the leucine content is higher than 5%, or higher than 10%, or higher than 15%, or higher than 17% of the total protein content based on weight.

11. The composition, comprising EPA and/or DPA for use according to any of claims 7 to 10, wherein the protein comprises at least one protein from a protein source selected from the group consisting of casein, caseinate, soy and wheat.

12. The composition, comprising EPA and/or DPA for use according to any of claims 7 to 11, wherein the protein comprises at least 15wt%, preferably at least 25wt% of whey based on the total protein content.

13. The composition, comprising EPA and/or DPA for use according to any of claims 5 to 12, further comprising a dietary fibre, a vitamin, a mineral, a trace element, β-carotenoid, a flavonoid, a nucleotide, L-carnitin, choline, inositol, taurine, creatine, and/or a co-enzyme.

14. The composition, comprising EPA and/or DPA for use according to any of claims 5 to 13, wherein the alkylating drug is selected from the group consisting of cisplatin, oxaliplatin, cyclophosphamid, ifosfamid, trofosfamid, melphalan, chlorambucil, estramustin, busulfan, treosulfan, carmustin, lomustin, nimustin, streptozocin, procarbazin, dacarbazin, temozolomid, and thiotepa; the antimetabolite is selected from the group consisting of 5-fluorouracil, methotrexate, azacitidin, capecitabin, doxifluridin, cytarabin, gemcitabin, 6-thioguanin, pentostatin, azathioprin, 6-mercaptopurin, fludarabin, and cladribin; the antimytotic cytostatic is selected from the group consisting of vinorelbin, vincristin, vinblastin, and vindesin; the topoisomerase inhibitor is selected from the group consisting of doxorubicin, camptothecin, topotecan, irinotecan, etoposid, and teniposid; the antitumor antibiotic is selected from the group consisting of tamoxifen, 5-fluoro-5'-deoxyuridine, belomycin, actinomycin D, and mitomycin; and/or the cytostatic is selected from the group consisting of L-asparaginase, hydroxycarbamide, mitotan, amatoxine, and altretamin.

15. The composition, comprising EPA and/or DPA for use according to any of claims 5 to 14, wherein said composition is a pharmaceutical or a nutritional composition.

16. EPA and/or DPA for use according to any of claims 1 to 4, or the composition, comprising EPA and/or DPA for use according to any of claims 5 to 14, wherein the neoplastic disease is a benign or malign tumor.

17. EPA and/or DPA for use according to any of claims 1 to 4, or the composition, comprising EPA and/or DPA for use according to any of claims 5 to 14, wherein the neoplastic disease is selected from the group consisting of pancreatic cancer, melanoma, brain tumor, bladder cancer, breast cancer, bladder cancer, ovary cancer, prostate cancer, colon cancer, gastric cancer, endometrial cancer, sarcoma, blood born tumor, leukemia, blastoma, glioma, mesothelioma, neuroblastoma, kidney cancer, liver cancer, lung cancer, and melanoma.

18. EPA and/or DPA for use according to any of claims 1 to 4, or the composition comprising EPA and/or DPA for use according to any of claims 5 to 14, wherein said EPA and/or DPA or said composition is/are administered prior to the chemotherapeutic agent, in parallel with the chemotherapeutic agent, or subsequent to the chemotherapeutic agent, and/or radiotherapy, preferably administered prior to the chemotherapeutic agent.

19. EPA and/or DPA for use according to any of claims 1 to 4, or the composition comprising EPA and/or DPA for use according to any of claims 5 to 14, wherein said EPA and/or DPA or said composition is/are administered 1 to 3 days, preferably 2 to 5, more preferably 3 to 7 days before the chemotherapeutic agent, even more preferably more than one week, preferably on a daily basis.

20. EPA and/or DPA for use according to any of claims 1 to 4, or the composition, comprising EPA and/or DPA for use according to any of claims 5 to 14, wherein said EPA and/or DPA or said composition is/are administered 1 to 3 days, preferably 2 to 5, more preferably 3 to 7 days before the chemotherapeutic agent, and either after or between the treatment(s) with the chemotherapeutic agent.

21. EPA and/or DPA for use according to any of claims 1 to 4, or the composition, comprising EPA and/or DPA for use according to any of claims 5 to 14, wherein said EPA and/or DPA or said composition is/are administered in form of a powder, a tablet, a capsule, or a fluid.

## Patentansprüche

1. EPA und/oder DPA zur Verwendung zur Verminderung von Nebenwirkungen eines chemotherapeutischen Wirkstoffs zur Verwendung bei der Behandlung einer neoplastischen Krankheit, der chemotherapeutische Wirkstoff ausgewählt aus der Gruppe, bestehend aus einem alkylierenden Medikament, einem Antimetaboliten, einem antimyotischen Zytostatikum, einem Topoisomerase-Hemmer, einem Antitumor-Antibiotikum und einem beliebigen anderen Zytostatikum und/oder Strahlentherapie, wobei die EPA und/oder DPA Teil einer Ernährungszusammensetzung sind, die einen Proteingehalt von mehr als 10 % und einen Leucingehalt von mehr als 5 % des Gesamtproteingehalts basierend auf Gewicht hat.

2. EPA und/oder DPA zur Verwendung nach Anspruch 1, wobei die EPA und/oder DPA ferner zur Verwendung zum Verbessern der Wirkung des chemotherapeutischen Wirkstoffs ist, wobei der chemotherapeutische Wirkstoff höhere Toxizität an Tumorzellen und parallel dazu niedrigere Toxizität an gesunden Nichttumorzellen aufweist.

3. EPA und/oder DPA zur Verwendung nach Anspruch 1 oder 2, wobei EPA einem Patienten in einer Menge von 1 bis 4000 mg pro Tag, bevorzugt 3 bis 3000 mg pro Tag, bevorzugter 2 bis 2000 mg pro Tag verabreicht wird.

4. EPA und/oder DPA zur Verwendung nach einem der Ansprüche 1-3, wobei DPA einem Patienten in einer Menge von mehr als 50 mg pro Tag, bevorzugt mehr als 100 mg pro Tag, bevorzugt mehr als 150 mg pro Tag, bevorzugter 200 bis 4000 mg/Tag, noch bevorzugter 250 bis 3200 mg/Tag, sogar noch bevorzugter 300 bis 2000 mg/Tag verabreicht wird.

5. Zusammensetzung, umfassend EPA und/oder DPA zur Verwendung nach Anspruch 1 oder 2, wobei EPA in einer Menge von 1 bis 1000 mg/100 ml, bevorzugt 1 bis 700 mg/100 ml, ist und/oder DPA in einer Menge von mehr als 50 mg/100 ml, ist.

6. Zusammensetzung, umfassend EPA und/oder DPA zur Verwendung nach Anspruch 5, wobei DPA in einer Menge von 50 bis 1000 mg/100 ml, bevorzugt 65 bis 800 mg/100 ml, bevorzugter 80 bis 500 mg/100 ml, ist.

7. Zusammensetzung, umfassend EPA und/oder DPA zur Verwendung nach Anspruch 5 oder 6, ferner umfassend ein Protein, ein Kohlehydrat, ein Lipid, eine Aminosäure, bevorzugt Leucin, und/oder eine Fettsäure.

8. Zusammensetzung, umfassend EPA und/oder DPA zur Verwendung nach Anspruch 7, wobei das zusätzliche Lipid in einem Gehalt von mehr als 10 % der Summe von EPA und DPA, basierend auf Gewicht, ist und DPA in einem Gehalt von mehr als 5 % ist, bevorzugt mehr als 10 %, bevorzugter mehr als 20 % der gesamten EPA und DPA.

9. Zusammensetzung, umfassend EPA und/oder DPA zur Verwendung nach Anspruch 7 oder 8, wobei das zusätzliche Lipid oder die zusätzliche Fettsäure ausgewählt ist aus der Gruppe, bestehend aus Buttersäure, Capronsäure, Caprylsäure, Caprinsäure, Laurinsäure, Myristinsäure, Palmsäure, Palmitat, Palmitoleinsäure, Stearinsäure, Ölsäure, Linolsäure, Alphalinolensäure, Gammalinolensäure, Arachinsäure, Eicosanoidsäure, Gammalinolensäure, Arachidonsäure, Behensäure, Erucasäure, Lignocerinsäure und Nervonsäure.

10. Zusammensetzung, umfassend EPA und/oder DPA zur Verwendung nach einem der Ansprüche 7 bis 9, wobei der Proteingehalt höher als 10 n-% oder höher als 15 n-% oder höher als 20 n-% ist und der Leucingehalt höher als 5 % oder höher als 10 % oder höher als 15 % oder höher als 17 % des Gesamtproteingehalts, basierend auf Gewicht, ist.

11. Zusammensetzung, umfassend EPA und/oder DPA zur Verwendung nach einem der Ansprüche 7 bis 10, wobei das Protein mindestens ein Protein aus einer Proteinquelle, ausgewählt aus der Gruppe, bestehend aus Casein, Caseinat, Soja und Weizen, umfasst.

12. Zusammensetzung, umfassend EPA und/oder DPA zur Verwendung nach einem der Ansprüche 7 bis 11, wobei das Protein mindestens 15 Gew.-% bevorzugt mindestens 25 Gew.-% von Molke, basierend auf dem Gesamtproteingehalt, umfasst.

13. Zusammensetzung, umfassend EPA und/oder DPA zur Verwendung nach einem der Ansprüche 5 bis 12, umfassend eine Diätfaser, ein Vitamin, ein Mineral, ein Spurenelement, β-Carotenoid, ein Flavonoid, ein Nukleotid, L-Carnitin, Cholin, Inositol, Taurin, Creatin und/oder ein Coenzym.

14. Zusammensetzung, umfassend EPA und/oder DPA zur Verwendung nach einem der Ansprüche 5 bis 13, wobei das alkylierende Medikament ausgewählt ist aus der Gruppe, bestehend aus Cisplatin, Oxaliplatin, Cyclophosphamid, Ifosfamid, Trofosfamid, Melphalan, Chlorambucil, Estramustin, Busulfan, Treosulfan, Carmustin, Lomustin, Nimustin, Streptozocin, Procarbazin, Dacarbazin, Temozolomid und Thiotepa; der Antimetabolit ausgewählt ist aus der Gruppe, bestehend aus 5-Fluoruracil, Methotrexat, Azacitidin, Capecitabin, Doxifluridin, Cytarabin, Gemeitabin, 6-Thioguanin, Pentostatin, Azathioprin, 6-Mercaptopurin, Fludarabin, und Cladribin; das antimyotische Cytostaticum ausgewählt ist aus der Gruppe, bestehend aus Vinorelbin, Vincristin, Vinblastin und Vindesin; der Topoimerase-Hemmer ausgewählt ist aus der Gruppe, bestehend aus Doxorubicin, Camptothecin, Topotecan, Irinotecan, Etoposid und Teniposid; das Antitumor-Antibiotikum ausgewählt ist aus der Gruppe, bestehend aus Tamoxifen, 5-Fluor-5'Deoxyuridin, Belomycin, Actinomycin D und Mitomycin; und/oder das Cytostaticum ausgewählt ist aus der Gruppe, bestehend aus L-Asparaginase, Hydroxycarbamid, Mitotan, Amatoxin und Altretamin.

15. Zusammensetzung, umfassend EPA und/oder DPA zur Verwendung nach einem der Ansprüche 5 bis 14, wobei die Zusammensetzung ein Pharmazeutikum oder eine Ernährungszusammensetzung ist.

16. EPA und/oder DPA zur Verwendung nach einem der Ansprüche 1 bis 4 oder Zusammensetzung, umfassend EPA und/oder DPA zur Verwendung nach einem der Ansprüche 5 bis 14, wobei die neoplastische Krankheit ein gutartiger oder bösartiger Tumor ist.

17. EPA und/oder DPA zur Verwendung nach einem der Ansprüche 1 bis 4 oder Zusammensetzung, umfassend EPA und/oder DPA zur Verwendung nach einem der Ansprüche 5 bis 14, wobei die neoplastische Krankheit ausgewählt ist aus der Gruppe, bestehend aus Bauchspeicheldrüsenkrebs, Melanom, Gehirntumor, Blasenkrebs, Brustkrebs, Blasenkrebs, Eierstockkrebs, Prostatakrebs, Darmkrebs, Magenkrebs, Gebärmutterschleimhautkrebs, Sarkom, durch Blut übertragener Tumor, Leukämie, Blastom, Gliom, Mesotheliom, Neuroblastom, Nierenkrebs, Leberkrebs, Lungenkrebs und Melanom.

18. EPA und/oder DPA zur Verwendung nach einem der Ansprüche 1 bis 4 oder Zusammensetzung, umfassend EPA und/oder DPA zur Verwendung nach einem der Ansprüche 5 bis 14, wobei die EPA und/oder DPA oder die Zusammensetzung vor dem chemotherapeutischen Wirkstoff, parallel mit dem chemotherapeutischen Wirkstoff oder anschließend an den chemotherapeutischen Wirkstoff und/oder Strahlentherapie verabreicht wird/werden, bevorzugt verabreicht vor dem chemotherapeutischen Wirkstoff.

19. EPA und/oder DPA zur Verwendung nach einem der Ansprüche 1 bis 4 oder Zusammensetzung, umfassend EPA und/oder DPA zur Verwendung nach einem der Ansprüche 5 bis 14, wobei die EPA und/oder DPA oder die Zusammensetzung 1 bis 3 Tage, bevorzugt 2 bis 5, bevorzugter 3 bis 7 Tage vor dem chemotherapeutischen Wirkstoff verabreicht wird/werden, noch bevorzugter mehr als eine Woche, bevorzugt auf täglicher Basis.

20. EPA und/oder DPA zur Verwendung nach einem der Ansprüche 1 bis 4 oder Zusammensetzung, umfassend EPA und/oder DPA zur Verwendung nach einem der Ansprüche 5 bis 14, wobei die EPA und/oder DPA oder die Zusammensetzung 1 bis 3 Tage, bevorzugt 2 bis 5, bevorzugter 3 bis 7 Tage vor dem chemotherapeutischen Wirkstoff und entweder nach oder zwischen der/den Behandlung/en mit dem chemotherapeutischen Wirkstoff verabreicht wird/werden.

21. EPA und/oder DPA zur Verwendung nach einem der Ansprüche 1 bis 4 oder Zusammensetzung, umfassend EPA und/oder DPA zur Verwendung nach einem der Ansprüche 5 bis 14, wobei die EPA und/oder DPA oder die Zusammensetzung in Form eines Pulvers, einer Tablette, einer Kapsel oder einer Flüssigkeit verabreicht wird/werden.

## Revendications

1. EPA et/ou DPA pour une utilisation dans la réduction d'effets secondaires d'un agent de chimiothérapie pour une utilisation dans le traitement d'une maladie néoplastique, ledit agent de chimiothérapie étant choisi dans le groupe consistant en un médicament alkylant, un anti-métabolite, un cytostatique antimytotique, un inhibiteur de topoisomérase, un antibiotique anti-tumoral, et tout autre cytostatique, et/ou de radiothérapie, dans lequel l'EPA et/ou DPA sont une partie d'une composition nutritionnelle ayant une teneur en protéine supérieure à 10 % en, et une teneur en leucine supérieure à 5 % de la teneur totale en protéine sur la base de la masse.

2. EPA et/ou DPA pour une utilisation selon la revendication 1, dans lequel l'EPA et/ou DPA est de plus destiné à une utilisation pour promouvoir l'effet de l'agent de chimiothérapie, sur quoi l'agent de chimiothérapie présente une toxicité plus élevée sur des cellules tumorales et en parallèle une toxicité plus faible sur des cellules non-tumorales saines.

3. EPA et/ou DPA pour une utilisation selon la revendication 1 ou 2 ; dans lequel EPA est administré à un sujet dans une quantité de 1 à 4 000 mg par jour, de préférence de 3 à 3 000 mg par jour, encore mieux de 2 à 2 000 mg par jour.

4. EPA et/ou DPA pour une utilisation selon l'une quelconque des revendications 1-3, dans lequel DPA est administré à un sujet dans une quantité supérieure à 50 mg par jour, de préférence supérieure à 100 mg par jour, encore mieux supérieure à 150 mg par jour, bien mieux encore de 200 à 4 000 mg/jour, particulièrement de préférence de 250 à 3 200mg/jour et encore mieux de préférence de 300 à 2 000 mg/jour.

5. Composition, comprenant EPA et/ou DPA pour une utilisation selon la revendication 1 ou 2 ; dans laquelle EPA est dans une quantité de 1 à 1 000 mg/100 ml, de préférence de 1 à 700 mg/100 ml et/ou DPA est dans une quantité supérieure à 50 mg/100 ml.

6. Composition, comprenant EPA et/ou DPA pour une utilisation selon la revendication 5, dans laquelle DPA est dans une quantité de 50 à 1 000 mg/100 ml, de préférence de 65 à 800 mg/100 ml, encore mieux de 80 à 500 mg/100 ml.

7. Composition, comprenant EPA et/ou DPA pour une utilisation selon la revendication 5 ou 6, comprenant de plus une protéine, un hydrate de carbone, un lipide, un acide animé, de préférence la leucine, et/ou un acide gras.

8. Composition, comprenant EPA et/ou DPA pour une utilisation selon la revendication 7, dans laquelle le lipide supplémentaire est dans une teneur supérieure à 10 % de la somme d'EPA et DPA sur la base de la masse et DPA est dans une teneur supérieure à 5 %, de préférence supérieure à 10 %, encore mieux supérieure à 20 % du total d'EPA et DPA.

9. Composition, comprenant EPA et/ou DPA pour une utilisation selon la revendication 7 ou 8, dans laquelle le lipide supplémentaire ou acide gras supplémentaire est choisi dans le groupe consistant en acide butyrique, acide caproïque, acide caprylique, acide caprique, acide laurique, acide myristique, acide palmitique, palmitate, acide palmitoléique, acide stéarique, acide oléique, acide linoléique, acide alpha linoléique, acide gamma linoléique, acide arachidique, acide eicosaénoïque, acide dihomo gamma-linolénique, acide arachidonique, acide béhénique, acide érucique, acide lignocérique et acide nervoïque.

10. Composition, comprenant EPA et/ou DPA pour une utilisation selon l'une quelconque des revendications 7 à 9, dans laquelle la teneur en protéine est supérieure à 10 % en, ou supérieure à 15 % en, ou supérieure à 20 % en, et la teneur en leucine est supérieure à 5 %, ou supérieure à 10 %, ou supérieure à 15 %, ou supérieure à 17 % de la teneur totale en protéine sur la base de la masse.

11. Composition, comprenant EPA et/ou DPA pour une utilisation selon l'une quelconque des revendications 7 à 10, dans laquelle la protéine comprend au moins une protéine parmi une source de protéine choisie dans le groupe consistant en caséine, caséinate, soja, ou blé.

12. Composition, comprenant EPA et/ou DPA pour une utilisation selon l'une quelconque des revendications 7 à 11, dans laquelle la protéine comprend au moins 15 % en masse, de préférence au moins 25 % en masse de blé sur la base de la teneur totale en protéine.

13. Composition, comprenant EPA et/ou DPA pour une utilisation selon l'une quelconque des revendications 5 à 12, comprenant de plus une fibre alimentaire, une vitamine, un minéral, un élément de trace, un β-caroténoïde, un flavonoïde, un nucléotide, la L-carnitine, la choline, l'inositol, la taurine, la créatine, et/ou une co-enzyme.

14. Composition comprenant EPA et/ou DPA pour une utilisation selon l'une quelconque des revendications 5 à 13, dans laquelle le médicament alkylant est choisi dans le groupe consistant en cisplatine, oxaliplatine, cyclophosphamide, ifosfamide, trofosfamide, melphalan, chlorambucil, estramustine, busulfan, tréosulfan, carmustine, lomustine, nimustine, streptozocine, procarbazine, dacarbazine, témozolomide, et thiotépa ; l'antimétabolite est choisi dans le groupe consistant en 5-fluorouracil, méthotréxate, azacitidine, capécitabine, doxifluridine, cytarabine, gemcitabine, 6-thioguanine, pentostatine, azathioprine, 6-mercaptopurine, fludarabine, et cladribine ; le cytostatique anti-mytotique est choisi dans le groupe consistant en vinorelbine, vincristine, vinblastine, et vindésine ; l'inhibiteur de topoisomérase est choisi dans le groupe consistant en doxorubicine, camptothécine, topotécan, irinotécan, étoposide, et téniposide ; l'antibiotique antitumoral est choisi dans le groupe consistant en tamoxifène, 5-fluoro-5'-déoxyuridine, bélomycine, actinomycine D, et mitomycine ; et/ou le cytostatique est choisi dans le groupe consistant en L-asparaginase, hydroxycarbamide, mitotan, amatoxine, et altrétamine.

15. Composition, comprenant EPA et/ou DPA pour une utilisation selon l'une quelconque des revendications 5 à 14, dans laquelle ladite composition est un produit pharmaceutique ou une composition nutritionnelle.

16. EPA et/ou DPA pour une utilisation selon l'une quelconque des revendications 1 à 4, ou composition, comprenant EPA et/ou DPA pour une utilisation selon l'une quelconque des revendications 5 à 14, dans laquelle la maladie néoplastique est une tumeur bénigne ou maligne.

17. EPA et/ou DPA pour une utilisation selon l'une quelconque des revendications 1 à 4, ou composition, comprenant EPA et/ou DPA pour une utilisation selon l'une quelconque des revendications 5 à 14, dans lequel la maladie néoplastique est choisie dans le groupe consistant en cancer du pancréas, mélanome, tumeur du cerveau, cancer de la vessie, cancer du sein, cancer de la vessie, cancer de l'ovaire, cancer de la prostate, cancer du côlon, cancer gastrique, cancer de l'endomètre, sarcome, tumeur à diffusion hématogène, leucémie, blastome, gliome, mésothéliome, neuroblastome, cancer du rein, cancer du foie, cancer du poumon, et mélanome.

18. EPA et/ou DPA pour une utilisation selon l'une quelconque des revendications 1 à 4, ou composition comprenant EPA et/ou DPA pour une utilisation selon l'une quelconque des revendications 5 à 14, dans lequel ledit EPA et/ou DPA ou ladite composition est/sont administrés avant l'agent de chimiothérapie, en parallèle avec l'agent de chimiothérapie, ou après l'agent de chimiothérapie, et/ou la radiothérapie, de préférence administrés avant l'agent de chimiothérapie.

19. EPA et/ou DPA pour une utilisation selon l'une quelconque des revendications 1 ou 4, ou composition comprenant EPA et/ou DPA pour une utilisation selon l'une quelconque des revendications 5 à 14, dans lequel ledit EPA et/ou DPA ou ladite composition est/sont administrés 1 à 3 jours, de préférence 2 à 5, encore mieux 3 à 7 jours avant l'agent de chimiothérapie, bien mieux encore plus d'une semaine, de préférence sur une base journalière.

20. EPA et/ou DPA pour une utilisation selon l'une quelconque des revendications 1 à 4, ou composition, comprenant EPA et/ou DPA pour une utilisation selon l'une quelconque des revendications 5 à 14, dans lequel ledit EPA et/ou DPA ou ladite composition est/sont administrés 1 à 3 jours, de préférence 2 à 5, encore mieux 3 à 7 jours, avant l'agent de chimiothérapie, et soit après soit pendant le(s) traitement(s) avec l'agent de chimiothérapie.

21. EPA et/ou DPA pour une utilisation selon l'une quelconque des revendications 1 à 4, ou composition, comprenant EPA et/ou DPA pour une utilisation selon l'une quelconque des revendications 5 à 14, dans lequel ledit EPA et/ou DPA ou ladite composition est/sont administrés dans une forme d'une poudre, d'un comprimé, d'une capsule, ou d'un fluide.
